(19) Europäisches Patentamt
European Patent Office
Office européen des brevets

(11) Publication number : **0 455 225 B1**

(12) **EUROPEAN PATENT SPECIFICATION**

(45) Date of publication of patent specification :
29.03.95 Bulletin 95/13

(51) Int. Cl.$^6$ : **G01N 33/68**, G01N 33/72

(21) Application number : **91107023.3**

(22) Date of filing : **30.04.91**

(54) **Method for measuring the percentage of glycation of a particular protein.**

(30) Priority : **01.05.90 JP 115547/90**

(43) Date of publication of application :
**06.11.91 Bulletin 91/45**

(45) Publication of the grant of the patent :
**29.03.95 Bulletin 95/13**

(84) Designated Contracting States :
**DE FR GB IT NL**

(56) References cited :
**US-A- 4 269 605**
**US-A- 4 861 728**

(73) Proprietor : **NACALAI TESQUE, INC.**
**498, Higashi tamaya-cho,**
**Nijo Karasume,**
**Nakagyo-ku**
**Kyoto-shi, Kyoto-fu, 604 (JP)**

(72) Inventor : **Miyake, Takehiro**
**405, 25-16, Nishikanmuri 1-chome**
**Takatsuki-shi, Osaka-fu, 569 (JP)**
Inventor : **Tanaka, Kimikazu**
**7-18, Nishikonoike-cho 1-chome**
**Higashiosaka-shi, Osaka-fu, 578 (JP)**

(74) Representative : **Kador & Partner**
**Corneliusstrasse 15**
**D-80469 München (DE)**

## Description

### Background of the Invention

#### Field of the Invention

This invention relates to a method for measuring the percentage of glycation of a particular protein.

#### Prior Art

A complication that occurs in an advanced stage of diabetes decisivly influences the convalescence of the patient. Herefore, preventing the complication of this disease is most important for diabetes remedy.

It is reported that protein in the blood of a diabetes patient undergoes nonenzymatic glycation to increase nonenzymatically glycated protein in blood compared to the case of a healthy man, and the relation between nonenzymatically glycated protein and disease has been studied extensively (Biochem. Biophys. Res. Commun., 67, 103 (1975), J. Biol. Chem., 254,702 (1979), Proc. Natl. Acad. Sci. USA, 78, 2393 (1982), Diabetes, 31,283 (1982), Proc. Natl. Acad. Sci. USA, 75, 2918 (1978), J. Clinical Pathology, 37, 841 (1984), Annals of Clinical Biochemistry, 21, 2 (1984).

As index of control of blood sugar, nonenzymatically glycated hemoglobin which shows satisfactory correlation to the blood sugar level for the previous 1-2 months is thought to be clinically useful together with fasting blood sugar which reflects the current glycometabolism, and it is extensively adopted as an index of the diabetes remedy. However, blood sugar level is subject to within-day precision greatly, while nonenzymatically glycated hemoglobin takes long time until confirmation of effects of the remedy.

With an aim of quicker confirmation of the treatment effects, fructosamine which is a capacity of reduction of the nonenzymatically glycated protein in blood reflecting the status of blood sugar for the previous 1-2 weeks is measured and made use of for diabetes remedy. In this case, however, influence of coexistent reducing materials, e.g., bilirubin and L-ascorbic acid, is posing problems (J. Clin. Lab. Inst. Reag., Vol. 13, No. 1, page 87 (1990). As an index of short-term blood sugar control, nonenzymatically glycated albumin is noted, and extensive researches and investigations have been conducted to develop a simple method of measuring this material.

Further, nonenzymatically glycated protein is of either Schiff base derivative (unstable) or ketoamine adduct (stable) occurring as a result of Amadori rearrangement and stabilization of Schiff base ("Rinsho Kensa", Journal of Medfical Technology, Vol. 33, No. 8, page 893 (1989)). Further, it is becoming important to measure Amadori rearrangement products, which is not affected by short-term blood sugar changes and satisfactorily reflects blood sugar level for a fixed past time.

As conventional method of determining glycated protein, there are isoelectric focusing electrophoresis, cation-exchange chromatography, spectrophotometry, colorimetric assay, high-performance liquid chromatographic assay, mini-column method and affinity chromatography (Diabetologia, 31, 627-631 (1988), "Kensa to Gijutsu" (Modern Medical Laboratory), Vol. 14, No.11, page 1,155 (1986)), and the assay is clinically extensively used to measure glycated protein in sample (i.e., test sample solution).

None of the above methods, however, can be thought to be satisfactory in all such respects as sensitivity of measurment, operation control property and test sample treatment time, and there are difficiencies in practice which have to be improved. Thus, there has been a demand for establishment of a method, which permits assay of a large number of test samples easily, accurately and in a short period of time.

For solving the above problems, it has been thought to have report to application of immunological assay, and there are reports that determination of glycated protein by radioimmunoassay (RIA) is possible by obtaining antiserum or monoclonal antibody having peculiar affinity to glycitol lysine residue which is one of glycated locations of glycated protein (J. Clin. Invest., 72,1427 (1983), Jap. J. Clin. Path., Supplement to Vol. 33, page 226 (1985), J. Japan Diab. Soc., Vol. 29, page 581 (1986), Clinica. Chimica. Acta, 153, 293 (1986), J. Japan, Diab. Soc., Vol. 28, page 695 (1985)).

The above immunological methods all use anti-reduction type glycated protein antibody to Amadori rearrangement products of glycated protein, and are excellent methods as a method for measuring glycated protein. However, these methods have problems in that it is necessary to specifically separate and measure the total quantity of a particular glycated protein which has high significance to assay clinically (J. Japan Diab. Soc., Suppliement to Vol. 34, page 96 (1986)).

Phenylboronic acid affinity chromatography which is less influence to small variations of temperature, pH or ionic strength is an excellent method of separating glycated protein, and a kit for clinical use, which contains immobilized phenylboronic acid, has been developed by Amicon Corpooration and Pierce Chemical Company

2

EP 0 455 225 B1

etc. (Great Bretain Patent Application Disclosure No. 2,024,829 and US patent No. 4,269,605). These methods, however, have such problems as the necessity of a comparatively large amount of sample and that protein amount determination operation lacking in specificity is involved.

Further, there is a method, in which glycated protein is separated by using immobilized phenylboronic acid and a labeled antibody having specific affinity to a particular protein is used (Japanese Patent Application Laid Opened No. 100,660/1987). This method, however, has problems in that changes in percentages of various kinds of glycated protein are affected and it is necessary to measure separately the total amount of particular protein to determine the glycation percentage thereof.

Further, there is a method of glycated albumin assay based on changes of fluorescence wavelength using a fluorescence labeled boronic acid derivative as reagent for glycated albumin detection (Clin. Chem. Acta, 149, 13 (1985)). This method, however, is theoretically unsuited as a method for measuring of the percentage of glycation of a particular protein in that the reagent reacts with indefinite glycated protein, that the total amount of particular protein has to be measured separately and that the method has poor specificity to the particular protein in measuring method for protain.

To solve these problems, there has been proposed a method disclosed in Japanese Patent Application Laid Opened No. 16,964/1989, in which a particular protein is separated using an immobilized antibody and a monoclonal antibody capable of specifically recognizing glycated reduced site such as glycitol lysine or glycitol valine residue is used to measure the quantity of the particular protein or glycation percentage thereof. This method is an excellent method in that the glycation percentage can be measured without need of measuring the total amount of the particular protein. Hoowever, it has such problems as poverty of reproducibility or linearity of standard curve, that recognizable glycated site is constituted only by a portion of the overall glycated site and that 5 hours or more is required for measurement.

Further, there is a method, in which a boronic acid derivative labeled by a fluorescent dye is reacted with hemoglobin in sample, hemoglobin is captured using an antibody immobilized on a support, the total amount of hemoglobin is measured colorimetrically, and glycated hemoglobin is fluorometrically measured to determine the percentage of glycation of hemoglobin (US patent No. 4,861,728). This method, however, is not an economical method in that it requires a boronic acid derivative labeled by fluorescent dye exceeding the total amount of glycated hemoglobin existing in sample in great excess to the amount of antibody immobilized on a support. Besides, it has problems in operation control property, convenience and versatility in that two different measurements, i.e., colorimetry and fluorometry, are necessary for the glycation percentage determination.

Summary of the Invention

The present invention has been intended in the light of the drawbacks inherent in the prior art methods as discussed above, and its object is to provide an assay method, which is a combination of immunological techniques and an affinity adsorptive method and permits measuring the percentage of glycation of a particular protein in an easier operation, in a short period of time and accurately.

The inventors have conducted extensive researches and investigations with an aim of realizing easy method for measuring the percentage of glycation of a particular protein, and they found that the percentage of glycation of a particular protein in a sample can be easily determined by reacting the sample, i.e., test sample solution, with a solid phase of an antibody or active fragments thereof (i.e., part of the antibody in which antigen is recognized) insolbly affixed to a solid support, the antibody having a specific affinity to the particular protein to be measured, thereby capturing the particular protein to the solid phase, then separating liquid phase (i.e., sample) and solid phase, bringing about a reaction of a labeled boronic acid derivative having affinity to cis-diol group present on glycated site of the particular protein bound to the solid phase, thereby binding the labeling material to the glycated site, and measuring the amount of labeling material bound or not bound to the solid phase. The invention is predicated in this finding.

More specifically, according to the invention there is provided a method for measuring the percentage of glycation of a particular protein, which comprises the steps of contacting a sample containing the particular protein with a solid phase of an antibody or active fragments thereof affixed to a support, the antibody having a specific affinity to the particular protein, thereby selectively binding the particular protein to the solid phase, separating the sample and solid phase, then contacting a labeled boronic acid derivative having affinity to glycated site of protein to the separated solid phase, thereby binding a marker to glycated site of the particular protein bound to the solid phase, then separating solid and liquid phases of the reacted mixture, and measuring the amount of the marker in separated solid or liquid phase.

According to the invention, there is also provided the method for measuring the percentage of glycation of a particular protein as mentioned above, wherein the boronic acid derivative contains a dihydroxyboryl group.

3

According to the invention, there is further provided a method for measuring the percentage of glycation of a particular protein as mentioned above wherein a sample is selected from a group consisting of extracted urine, blood, plasma and serum.

According to the invention, there is further provided a method for measuring the percentage of glycation of a particular protein as mentioned above wherein the labeled boronic acid derivative is obtained by labeling with a member of a group consisting of an isotope, an enzyme, a coenzyme, a fluorescent dye, a chemiluminescent agent and a specific binding protein.

With the assay method according to the present invention, the boronic acid derivative can bind mainly Amadori rearrangement product (stable) among glycated protein, and thus there is no need of removal of Schiff base derivatives (unstable) of glycated protein by a pre-treatment of the sample (Clin. Chem., 28, 10, 2088-2094 (1982).

As shown above, according to the present invention the sole intended particular protein (such as albumin or hemoglobin, for instance) is separated specifically from a test sample solution. To this end, the particular protein is bound to a solid phase of an antibody or active fragments thereof having a specific affinity to the particular protein and affixed to a support through immuno-reaction of the antibody or active fragments thereof with respect to the particular protein, and then glycated protein in the bound particular protein is measured using a labeled boronic acid derivative (obtained by affixing an isotope, an enzyme or other labeling material to boronic acid derivative). Thus, it is possible to obtain efficient measurement of Amadori rearrangement product (which results from stabilization of Schiff base derivatives as glycated protein through Amadori rearrangement) without need of preparing any antibody that only have specific affinity to the glycated protein.

Brief Description of the Drawings

Figure 1 is a graph showing a standard curve representing the relation between absorbance based on labeling material amount determination and percentage of glycation of human serum albumin;

Figure 2 is an illustration showing a simplified model of an albumin molecule;

Figure 3 is an illustration showing a simplified model of a glycated albumin molecule;

Figure 4 is an illustration showing a simplified model of anti-albumin antibody bound to a support and capturing albumin irrespective of whether albumin is glycated or not; and

Figure 5 is an illustration showing a simplified model of labeled boronic acid derivative peculiarily bound only to glycated site of glycated albumin captured to the support shown in Figure 4.

Detailed Description of the Invention

Now, the invention will be described in detail. It is to be constructed that materials and material groups given below are by no means limitative.

The antibody used according to the invention may be derived in any way. For example, antiserum or ascites obtained from a mammal, e.g., mouse, rat or rabbit, immunized by administration of a particular protein or a refined one thereof as antigen may be used in situ or after refinement by a well-known method, e.g., salting-out method, gel filtration method, ion exchange chromatography, electrophoresis and affinity chromatography, to a polyclonal antibody. In addition, it is possible to use antibodies prepared by obtaining hybrid cells (hybridomas) from antibody product cells (such as spleen cells and lymph node cells) of mammals or the like sensitized by antigen and myeloma cells either in situ or after refinement in a well-known manner such as salting-out method or various chromatographic methods.

These antibodies may be used as their molecules themselves, or it is possible to use their active fragments (i.e., their part where antigen is recognized) such as Fab, Fab' or F(ab')2 obtained by their enzymatic treatment.

The shape of the solid support may be suitably selected depending on purpose. Examples of the shape are bead-like, test-plate-like, tubular, disc-like, spherical, stick-like and latex-like. The material of the support may be those used for supports for ordinary enzymeimmunoassay (EIA). Examples are glass, polysaccharides (e.g., cellulose, dextran, starch and dextrin) or derivatives thereof, silica gel, porous ceramics, metal oxides and various synthetic resins (e.g., polymers or copolymers from such as propyrene, vinyl chloride, vinyl acetate, vinyl propionate, acrylic acid, acryl ester, methacrylic acid, methacryl ester, styrene, methylstyrene, butadiene, isoprene, acrylamide, acrylonitrile, methacrylonitrile and others). Those supports are used either in situ or after introduction of such reactive functional groups as sulfonyl group and amino group by well-known means.

The antibody may be affixed to the support in a manner like those for immobilized enzymes, e.g., physical adsorption method, covalent bond method and cross-linking method. (For example, see Ichiro Chibata "Immobilized Enzyme" issued March 20, 1975 by Kodansha Company, pages 9 to 75.)

The boronic acid derivative may be of any structure having a dihydroxyboryl group. Examples are 1-amino-2-phenylethyl boronic acid, 3-[(aminocarbonyl)amino] phenyl boronic acid, 3-aminophenyl boronic acid, 3-(hydroxy)phenyl boronic acid, 2-(hydroxyamino)phenyl bronic acid and hydrochloride of 4-(hydroxyamino)phenyl boronic acid. Boronic acid derivatives having amino group are particularly suitable.

As linkage reagent for binding boronic acid derivative and labeling material to obtain labeled boronic acid derivative may be used glutaraldehyde, periodic acid, maleimide compounds (e.g., N-succinimidyl-2-maleimidoacetate, N-succinimidyl-4-maleimidobutyrate, N-succinimidyl-4-(N-maleimidomethyl)-cyclohexane-1-carboxylate and N-sulfosuccinimidyl-4-(N-maleimidomethyl)-cyclohexane-1-carboxylate), and dimaleimide compounds (e.g., N, N'-oxydimethylenedimaleimide and N,N'-o-phenylenedimaleimide).

As the labeling material for labeling boronic acid derivative may be used those frequently used for immunological assay and capable of being comparatively readily detected by some or other ways. Specific examples of the material are isotopes, enzymes (e.g., β-galactosidase, peroxidase, alkaline phosphatase, glucose oxidase and glucose-6-phosphate dehydrogenase), substrates of enzymes, fluorescent dyes (e.g., Fluorescein derivatives, Rhodamine derivatives and Umbelliferone), chemical luminescent agents (e.g., luminol derivatives and isoluminol derivatives), coenzyme and prosthetic group (e.g.,biotin, nicotinamide adenine dinucleotide) and specific binding proteins (e.g., avidin), the term "specific binding" meaning a character of binding to only specific compounds.

For the labeling using an isotope a method may be used, which conforms to "Jap. J. Clin. Path.", Special Issue No. 53, pages 24 to 25 (1983), issued on February 28, 1983 by Kanahara Shuppan, or to "Kensa to Gijutsu", Special Vol. 16, No. 7, pages 581 to 582 (1988), issued on June 15, 1988 by Igaku Shoin. For other labeling method may be used, which conforms to Eiji Ishikawa, Tadashi Kawakami and Kiyoshi Miyai "Enzymeimmunoassay", No. 3, pages 75 to 151, issued on May 15, 1987 by Igaku Shoin, or to Ichiro Chibata "Immobilized Enzyme", pages 9 to 75, issued March 20, 1975 by Kodansha Company.

The sample (i.e., test sample solution) to be assayed is not limited so long as it requires measurement of glycated protein. Examples are those derived from living organism and containing glycated protein, e.g., urea, blood, plasma and serum, or mixtures of protein and sugar, e.g., glucose and glucose-6-phospate. Glycated protein is described in "Jap. J. Clin. Path.", Vol. 33, No. 8, pages 879 to 899 (1989).

For measuring the percentage of glycation of a particular protein in a sample, it is important to use a sample, which can contain the particular protein at least at a concentration sufficient for the binding of the particular protein to all of a fixed amount of an antibody or active fragments thereof affixed to a support.

For binding the labeled boronic acid derivative to glycated site of glycated protein, usually the former is suitably used in the form of solution with pH 8 to 9 obtained by using ammonium acetate buffer solution, morpholine hydrochloric acid buffer solution, 2-amino-2-methylpropane,1,3-diol hydrochloric acid buffer solution, etc.

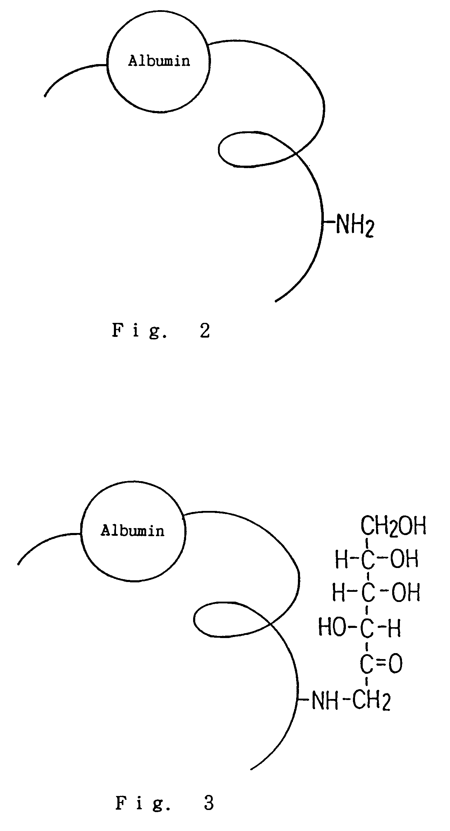

To facilitate the understanding of the invention, simplified models of reaction of albumin as particular protein are shown in Figures 2 to 5 as an example.

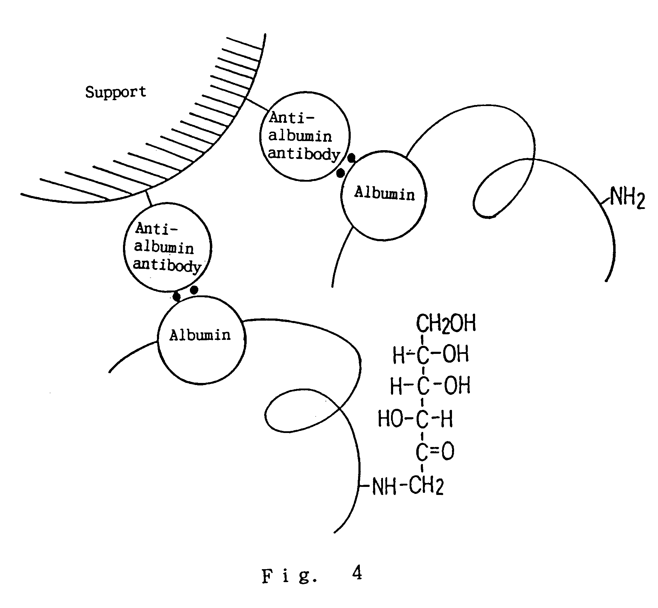

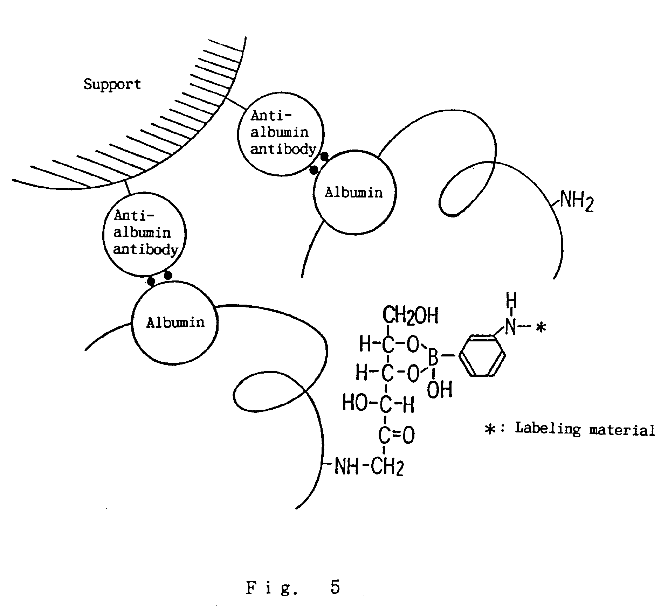

Figure 2 shows a model of an albumin molecule. Indicated at -NH2 is an amino group of albumin. Figure 3 shows glycated albumin (resulting from Amadori rearrangement which sugar bindes to the amino group of albumin shown in Figure 3). Figure 4 shows a state in which the anti-albumin antibody affixed to a support captures alubmin irrespective of whether albumin is glycated or not. Figure 5 shows a state, in which a labeled boronic acid derivative is peculiarly bound to sole glycated site of the captured glycated albumin shown in Figure 4. Indicated by the asterisk in Figure 5 is a labeling material.

With the method according to the invention, the quantity of the particular protein bound to the solid phase is a fixed quantity because of the use of a sample which contains the particular protein at least at a concentration sufficient for the binding of the particular protein to all of a fixed amount of an antibody or active fragments thereof affixed to a support. Thus, with measurement of the amount of the labeling material bound to the glycated site of the particular protein, the percentage of glycation of the particular protein can be measured without need of separately measuring the total amount of the particular protein. In addition, the method according to the invention permits the measurment of the percentage of glycation of a particular protein more accurately, more conveniently and in a shorter period of time compared to the prior art method.

Examples of the invention are given below to facilitate the understanding of the invention without any sense of limiting the invention.

Example 1

(1) Preparation of nonenzymatic glycated albumin and nonglycated albumin

50 mg of human serum albumin (available from Sigma Chemical Company), 50 mg of D-glucose and 4 mg of sodium borohydride were dissolved in 10 mM (M being mol/l) phosphate buffer solution pH 8.0, and the resultant solution held at 37 °C for 10 days. This albumin was desalted in gel filtration chromatography with "Sephadex G-25" (available from Pharmacia Company, as gel filtration chromatographic medium) and 0.25 M ammonium acetate buffer solution pH 9.0 as elute, followed by affinity chromatography using an absorbent with 3-aminophenylboronic acid (available from Aldrich Chemical Company, Inc.) bound thereto with 0.25 M ammonium acetate buffer solution pH 9.0 and 0.25 M acetate buffer solution pH 4.0 as elute. Albumin extract with elute 0.25 M ammonium acetate buffer solution pH 9.0 was separated as a non-glycated albumin fraction, while albumin extract with 0.25 M acetic acid buffer solution pH 4.0 was separated as a nonenzymatically glycated albumin fraction. These fractions were refined.

(2) Preparation of peroxidase-labeled phenylboronic acid derivative

4 mg of peroxidase from horseradish (by Sigma Company), 16 mg of 3-aminophenylboronic acid and 0.05 % of glutaraldehyde were mixed, and reacted at room temperature for 48 hours. Then, 2 mg of sodium borohydride was added, and the system was left at room temperature for 3 hours. Then, the system was desalted in gel filtration chromatography with "Sephadex G-25" and 0.25 M ammonium acetate buffer solution pH 9.0 as elute. Then, using an adsorbent, to which nonenzymatically glycated albumin prepared in (1) was bound, affinity chromatography was carried out with 0.25 M ammonium acetate buffer solution pH 9.0 and 0.25 M acetic acid buffer solution pH 4.0 as elute. Peroxidase-labeled phenylboronic acid derivative extract with 0.25 M acetic acid buffer solution pH 9.0 was separated and purified.

(3) Preparation of support with affixed anti-human serum antibody

To render anti-human serum albumin antibody insoluble by affixing it on solid phase, i.e., polystyrene beads, polystyrene beads (with a diamewter of 6.35 mm) was impregnated with anti-human serum albumin antibody soliution (100 to 200 μg/ml, 10 mM phosphate buffer solution pH 7.3) and then left overnight at 4°C , followed by dipping in 0.1 % bovine serum albumin (BSA) and leaving overnight at 4°C, thus preparing support with affixed anti-human serum antibody (solid phase).

(4) Measuring of the percentage of glycation of human serum albumin

Nonglycated albumin and nonenzymatically glycated albumin were mixed, and samples with glycated albumin percentages of 10, 20, 40, 60, 80 and 100 % were prepared by setting the human serum albumin concentration to 3g/dl, the albumin concentration in serum of the normal healthy man.

20 μl of each sample was diluted with physiological salt solution to 100 times, thus obtaining a diluted sample.

500 μl of the diluted sample was distributed into a test tube, followed by heating in water bath at 37°C for 5 minutes. Then, the support with affixed anti-human serum albumin antibody was charged into the test tube and reacted for 5 minutes. Then, the support and liquid phase were separated, and the support was then washed with physiological salt solution and reacted with 400 μl of peroxidase-labeled phenylboronic acid derivative solution (0.25 M ammonium acetate buffer solutoin pH 9.0) at 37°C for 30 minutes. Then, the support was washed with 0.25 M ammonium acetate buffer solution pH 9.0 and reacted with 500 μl of 1 mg/ml o-phenylenediamine solution (containing 0.1 M phosphate citrate buffer solution pH 6.0 and 5 mM hydrogen peroxide) at 37 °C for 10 minutes. The reaction was stopped at 1 N sulfuric acid of 2.0 ml, and 492 nm absorbance was measured.

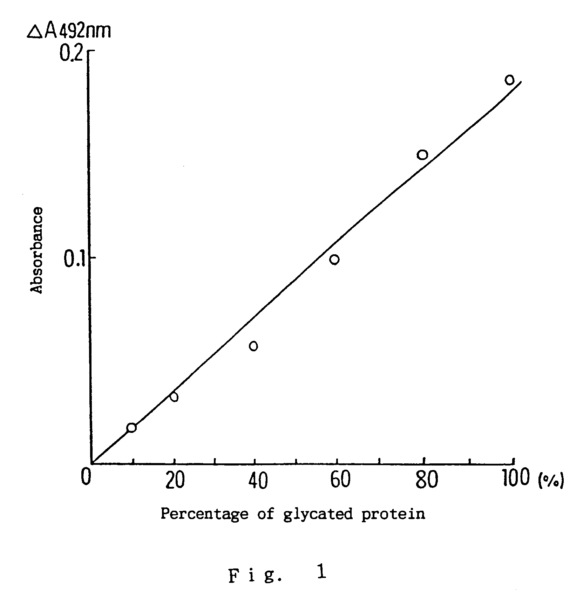

The results of measurement were shown in Figure 1. Indicated by $\Delta$A on the ordinate axis are values corrected by subtration of the absorbance of the blank value. As shown in Figure 1, a standard curve can be obtained, which represents a relation between absorbance and percentage of glycated protein. The absorbance was found to increase linearly in proportion to the increase of the glycated protein percentage, indicating a possibility of accurate measurment by the assay method according to the invention.

It was also found that time necessary for the assay was about one hour and that the necessary amount of sample was 20 μ l, indicating a possibility of processing of a large amount of samples in a short period of time and the necessity of very slight amount of sample.

Example 2

Electrophoresis separation of albumin in serum sample and measuring the percentage of glycation of extract albumin

(1) Electrophoresis separation of albumin

A serum sample was prepared by adding 5 g of D-glucose to 10 ml of controlled standard serum "Moni-Trol II.X" (by American Dade Company) and holding the resultant system at 37 °C for 2 days. A portion of the serum sample was electrophoresed with 10 (W/W %) polyacrylamide gel, and gel was then held dipped in a trichloroacetic acid aqueous solution for about one hour to cut out a portion corresponding to albumin. The cut-out gel was then poured into a test tube and washed until it became neutral with 0.1 M phosphate buffer solution pH 7.3. The gel was then mashed, and albumin was extracted by centrifugal separation.

(2) Measurement of the percentage of glycation of albumin

The percentage of glycation of albumin in the serum sample and albumin extracted from the electrophoretic gel was measured in the manner as in Example 1. The results of measurement are shown in Table 1 below.

## Table 1

| | Glycation percentage (%) |
|---|---|
| Albumin in serum | 9.5 |
| Albumin extracted from gel | 9.6 |

The percentage of glycation of albumin in the serum sample and that of albumin extracted from the gel were substantially equal. That is, measurment of the percentage of glycation of albumin in the sample serum could be obtained without being adversely affected by coexistent materials and similar to the case when albumin was solely separated by electrophoresis.

Example 3

Measuring the percentage of glycation of human serum albumin using avidin-labeled phenylboronic acid derivative and peroxidase-labeled biotin

(1) Preparation of avidin-labeled phenylboronic acid derivative

2 mg of avidin from egg white and 8 mg of 3-aminophenylboronic acid were dissolved in 2 ml of 0.5 M borate buffer solution pH 10.5, and 1 $\mu$l of 25 (W/W %) glutaraldehyde was added to the solution, followed by leaving at room temperature for 10 minutes. After 10 minutes, 500 $\mu$l of 0.25 M Tris-HCl buffer solution was added, and the reaction was stopped, followed by leaving still at room temperature for one hour. Avidin-labeled phenylboronic acid derivative was purified in the manner as in Example 1, and its pH was adjusted to 9.0 with 4N-sodium hydroxide.

(2) Measuring the percentage of glycation of human serum albumin

Human serum albumin samples with glycation percentages of 0, 50 and 100 % were prepared in the same way as in Example 1. The operation of measurement was carried out by using avidin-labeled phenylboronic acid derivative solution (0.25 M ammonium acetate buffer soluton pH 9.0) instead of peroxidase-labeled phenylboronic acid derivative and thus binding avidin to glycated site of glycated albumin. The support was washed with physiological salt solution (containing 0.05 (V/V %) polyoxyethylene sorbitan monolaurate) and reacted with a solution of peroxidase-labeled biotin (by Shigma Company) (2.7 U/ml 0.25 M ammonium acetate buffer solution pH 9.0) at 37°C for 10 minutes. Then the activity of enzyme was measured in the manner as in Example 1. The results are shown in Table 2 below.

Table 2

| Glycation percentage (%) | 50 | 100 |
|---|---|---|
| △A 492 nm | 0.864 | 1.812 |

The value was increased in proportion to increase of the glycation percentage (%). As shown, measurment of the percentage of glycation of human serum albumin using avidin-labeled phenylboronic derivative and peroxidase-labeled biotin could be possible by utilizing specific and selective property of avidin and biotin as peculiar binding protein.

Example 4

Measuring of the percentage of glycation of human hemoglobin

Nonenzymatically glycated human hemoglobin and nonglycated human hemoglobin were prepared, separated and purified in the manner as in Example 1. The percentrage of glycation was measureed in the manner as in Example 1 except for using a carrier with affixed anti-human hemoglobin antibody and peroxidase-labeled phenylboronic acid derivative solution (2 U/ml). The results of experiment are shown in Table 3.

Table 3

| Glycation percentage (%) | 25 | 50 | 75 | 100 |
|---|---|---|---|---|
| △A 492 nm | 0.128 | 0.466 | 0.647 | 0.899 |

It was found as a result of tests that measurement of the percentage of glycation of hemoglobin was possible as in the same of albumin in the manner as in Example 1.

Example 5

Measuring the percentage of glycation of slight albumin in urine

(1) Preparation of standard urine sample

Urine was deproteinized through an ultrafiltration membrane (by Toyo Roshi Company). Standard urine samples were prepared by adding nonenzymatically glycated albumin and nonglycated albumin obtained in Example 1 to the deproteinized urine.
The albumin concentration was set to 5 $\mu$g/ml, the albumin concentration in the urine of healthy man.

(2) Measuring the percentage of glycation of albumin in urine

As sample 400 $\mu$l of the urine sample was taken in a test tube, and 100 $\mu$l of 0.25 M ammonium acetate buffer solution pH 9.0 containing 2.5 (W/V %) sodium chloride and 2.0 (V/V) polyoxyethylene sorbitan monolaurate was added to the sample. The glycation percentage was measured in the manner as in Example 1 except for changing the antigen antibody reaction time from 5 minutes to 2 hours and also changing the enzymic reaction time from 10 minutes to 30 minutes. The results are shown in Table 4.

EP 0 455 225 B1

Table 4

Glycation percen-

| | tage (%) | | Sample 1 | Sample 2 |
|---|---|---|---|---|
| | 25 % | 50 % | | |
| $\triangle$A 492 nm | 0.298 | 0.529 | 0.021 | 0.043 |
| Glycation percentage (%) | --- | --- | 1.9 | 3.8 |

A standard curve could be formed from standard urine samples with glycation percentages of 0, 25 and 50 %. Also, the glycation percentage was determined with Samples 1 and 2 from urine of healthy man and found to range from about 2 to about 4 %.

As shown above, the method according to the invention permits measurement of the percentage of glycation of a particular protein in sample accurately, in short-term, conveniently and quickly compared to the prior art method.

In addition, with the method according to the invention by using a sample containing a particular protein at least at a concentration capable sufficient for the binding of all of a fixed amount of an antibody or active fragments thereof affixed to a support, a fixed amount of the particular protein is bound to the solid phase, and thus it is possible to determine the percentage of glycation of the particular protein without need of separately measuring the total amount of the particular protein but by measuring the amount of a marker bound to or not bound to glycated site of the particular protein with use of a labeled boronic acid derivative.

Further, the labeled boronic acid derivative necessary for the measurement need be present only in an amount in excess of the amount of particular protein that is bound to the solid phase, that is, there is not need of using an unnecessarily large amount of labeled boronic acid derivative, which is desired in view of economy.

**Claims**

1. A method for measuring the percentage of glycation of a particular protein, comprising the steps of contacting a sample containing said particular protein with a solid phase of an antibody or active fragments thereof affixed to a support, said antibody having a specific affinity to said particular protein, thereby selectively binding said particular protein to said solid phase, separating said sample and solid phase, contacting a labeled boronic acid derivative having affinity to glycated site of said particular protein, thereby binding a marker to said glycated site of said particular protein, then separating the solid and liquid phases of the reacted mixture, and measuring the amount of said marker in the solid or liquid phase.

2. The method according to claim 1, wherein said boronic acid derivative contains dihydroxyboryl group.

3. The method according to claims 1 or 2, wherein said sample is a member of a group consisting of urine, blood, plasma and serum.

4. The method according to claims 1 or 2, wherein said labeled boronic acid derivative is labeled by a member selected from the group consisting of isotopes, enzymes, coenzymes, fluorescent dyes, chemiluminescent agents and specific binding proteins.

**Patentansprüche**

1. Verfahren zur Bestimmung des Glykationsprozentsatzes eines bestimmten Proteins, das die Schritte umfaßt, daß eine Probe, die das bestimmte Protein enthält, mit einer festen Phase eines Antikörpers oder dessen aktiven Fragmentes, das an einem Träger haftet, in Kontakt gebracht wird, wobei der Antikörper eine spezifische Affinität zu dem bestimmten Protein aufweist, wodurch das bestimmte Protein an die feste Phase selektiv gebunden wird, die Probe und die feste Phase aufgetrennt werden, dann ein markiertes Boronsäurederivat mit Affinität zu der glykierten Stelle des Proteins in Kontakt gebracht wird, wodurch eine Markierungssubstanz auf die glykierte Stelle des bestimmten Proteins gebunden wird, die festen und flüssigen Phasen des umgesetzten Gemisches anschließend aufgetrennt werden und die Menge der Markierungssubstanz in der festen oder flüssigen Phase bestimmt wird.

2. Verfahren nach Anspruch 1, worin das Boronsäure-Derivat eine Dihydroxyborylgruppe enthält.

3. Verfahren nach Anspruch 1 oder 2, worin die Probe aus einer aus Urin, Blut, Plasma oder Serum bestehenden Gruppe stammt.

4. Verfahren nach Anspruch 1 oder 2, worin das markierte Boronsäure-Derivat mit einem Bestandteil, das aus der aus Isotopen, Enzymen, Coenzymen, Fluoreszenzfarbstoffen, Chemilumineszenzmitteln und spezifischen Bindungsproteinen bestehenden Gruppe ausgewählt ist, markiert ist.

## Revendications

1. Procédé de mesure du pourcentage de glycosylation d'une protéine particulière, selon lequel on met en contact un échantillon contenant cette protéine particulière, avec une phase solide comprenant un anticorps ou des fragments actifs de celui-ci, liés à un support, cet anticorps ayant une affinité spécifique pour la protéine particulière, de façon à lier sélectivement la protéine particulière à la phase solide, on sépare l'échantillon et la phase solide, on met en contact un dérivé d'acide borique marqué ayant une affinité pour un site glycosylé de la protéine particulière, de façon à lier un marqueur à ce site glycosylé de la protéine particulière, on sépare ensuite les phases solide et liquide du mélange ayant réagi, et on mesure la quantité de ce marqueur dans la phase solide ou liquide.

2. Procédé selon la revendication 1, dans lequel le dérivé d'acide borique contient un groupe dihydroxyboryle.

3. Procédé selon la revendication 1 ou 2, dans lequel l'échantillon est choisi parmi l'urine, le sang, le plasma et le sérum.

4. Procédé selon la revendication 1 ou 2, dans lequel le dérivé d'acide borique marqué, est marqué avec une substance choisie parmi les isotopes, les enzymes, les coenzymes, les colorants fluorescents, les agents chimioluminescents et les protéines de liaison spécifique.

Fig. 1

F i g.　2

F i g.　3

Support

Anti-
albumin
antibody

Anti-
albumin
antibody

Albumin

Albumin

$NH_2$

$CH_2OH$

$H-C-OH$

$H-C-OH$

$HO-C-H$

$C=O$

$NH-CH_2$

Fig. 4

F i g. 5